(19) Europäisches Patentamt
European Patent Office
Office européen des brevets

(11) Veröffentlichungsnummer: **0 484 660 A1**

# (12) EUROPÄISCHE PATENTANMELDUNG

(21) Anmeldenummer: **91115447.4**

(22) Anmeldetag: **12.09.91**

(51) Int. Cl.$^5$: **C07D 239/54**, A61K 31/505

(30) Priorität: **06.11.90 DE 4035599**

(43) Veröffentlichungstag der Anmeldung:
**13.05.92 Patentblatt 92/20**

(84) Benannte Vertragsstaaten:
**AT BE CH DE ES FR GB IT LI NL SE**

(71) Anmelder: **ARZNEIMITTELWERK DRESDEN GmbH**
**Wilhelm-Pieck-Strasse 35**
**O-8122 Radebeul(DE)**

(72) Erfinder: **Sauer, Wolfgang, Dr.**
**Platanenstrasse 39**
**W-0-8023 Dresden(DE)**
Erfinder: **Jänsch, Hans-Joachim, Dr.**
**Wilhelm-Pieck-Strasse 292**
**W-0-8122 Radebeul(DE)**
Erfinder: **Rostock, Angelika, Dr.**
**Böttgerstrasse 36**
**W-0-8023 Dresden(DE)**
Erfinder: **Faust, Gottfried, Dr.**
**Ernst-Thälmann-Strasse 53**
**W-0-8122 Radebeul(DE)**
Erfinder: **Lohmann, Dieter, Dr.**
**Hoflössnitzstrasse 30**
**W-0-8122 Radebeul(DE)**
Erfinder: **Bartsch, Reni, Dr.**
**Stephensonstrasse 11**
**W-0-8045 Dresden(DE)**
Erfinder: **Siegemund, Christine, Dipl.-Biol.**
**Hauptstrasse 12**
**W-0-8256 Weinböhla(DE)**

## (54) Neue 5-(Phenoxyalkanoylamino)-uracile, Verfahren zu ihrer Herstellung und ihre Verwendung als Arzneimittel.

(57) Die Efindung betrifft neue 5-(Phenoxyalkanoylamino)-uracile der Formel I

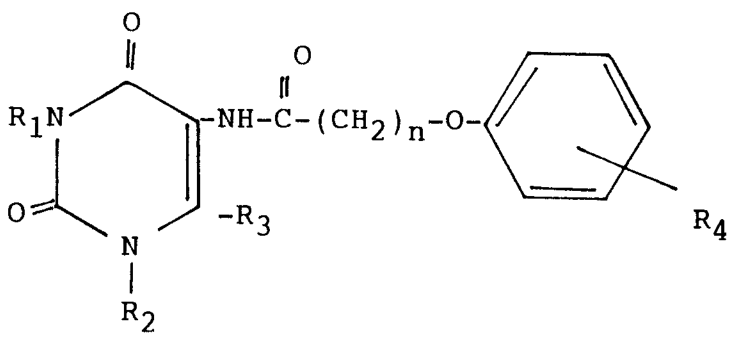

worin

$R_1$ und $R_2$ Wasserstoff, eine Alkylgruppe mit 1-6 C-Atomen, die geradkettig, verzweigt,gesättigt oder ungesättigt oder mit einer Hydroxy- bzw. Oxo-Gruppe substituiert sein kann, eine Cycloalkyl- oder Cycloalkylmethylgruppe, in welcher der Cycloalkylteil 3-7 C-Atome enthalten kann, eine Phenylalkylgruppe, in welcher der Alkylteil 1-3 C-Atome enthalten kann und der Phenylrest durch ein Halogenatom, eine Methoxy-, Methyl-oder Trifluormethylgruppe substituiert sein kann,

EP 0 484 660 A1

| | |
|---|---|
| $R_3$ | Wasserstoff, eine Methyl- oder Aminogruppe, |
| $R_4$ | Halogenatom, Methoxy- oder Methylgruppe, |
| n | 1,2 bedeuten kann, |

Verfahren zu ihrer Herstellung und ihre Verwendung als Arzneimittel.
Die Verbindungen der Formel I zeichnen sich durch eine starke cerebroprotektive Wirkung bei gleichzeitig hoher Verträglichkeit aus und eignen sich zur Behandlung von Hirnfunktionsstörungen.

Die Erfindung betrifft neue 5-(Phenoxyalkanoylamino)-uracile der allgemeinen Formel I

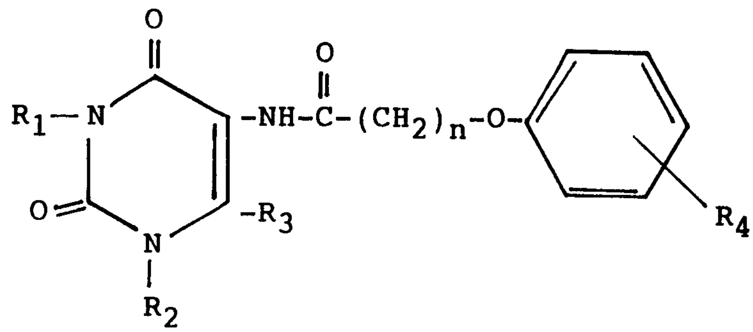

worin

| | |
|---|---|
| $R_1$ und $R_2$ | Wasserstoff, eine Alkylgruppe mit 1-6 C-Atomen, die geradkettig, verzweigt, gesättigt oder ungesättigt oder mit einer Hydroxy- bzw. Oxo-Gruppe substituiert sein kann, eine Cycloalkyl- oder Cycloalkylmethylgruppe, in welcher der Cycloalkylteil 3-7 C-Atome enthalten kann, eine Phenylalkylgruppe, in welcher der Alkylteil 1-3 C-Atome enthalten kann und der Phenylrest durch ein Halogenatom, eine Methoxy-, Methyl- oder Trifluormethylgruppe substituiert sein kann, |
| $R_3$ | Wasserstoff, eine Methyl- oder Aminogruppe, |
| $R_4$ | Halogenatom, Methoxy- oder Methylgruppe, |
| n | 1,2 bedeuten kann, |

Verfahren zu ihrer Herstellung, pharmazeutische Zubereitungen, die diese Verbindungen enthalten und ihre Verwendung als Arzneimittel.

Die erfindungsgemäßen neuen 5-(Phenoxyalkanoylamino)-uracile der allgemeinen Formel I zeichnen sich durch eine starke cerebroprotektive Wirkung bei gleichzeitig hoher Verträglichkeit aus, so daß sie in der Prophylaxe und Behandlung cerebraler Funktionsstörungen eingesetzt werden können.

Aus der Literatur ist u.a. das 6-Methyluracil bekannt (DD-PS A61K/264 182 8), sowie dessen zahlreichen biologischen Wirkungen. Als Beispiel sei die Wirkung als Antibiotika (Antibiotiki 24(1979),659) und als Strahlenschutzmittel (Arzneimittelforschung 27 (1977),132 ff) genannt.

In dem DD-PS A 61 K/264 182 8 wird die Verwendung des 6-Methyluracil als Nootropika beansprucht. Dieses Nootropikum wurde bisher noch nicht in die Praxis eingeführt.

Nootropika wie Piracetam und Meclofenoxat sind seit längerer Zeit in der medizinischen Praxis bekannt. Es besteht jedoch der Bedarf, Wirkstoffe zu finden, die eine erhöhte antiamnestische und antihypoxische Wirkung aufweisen und damit in der Lage sind, Hirnleistungen unter pathologischen Bedingungen zu normalisieren und das Hirn vor Sauerstoffmangel zu schützen.

Der Erfindung liegt die Aufgabe zugrunde, neue nootrop wirksame 5-(Phenoxyalkanoylamino)-uracile der allgemeinen Formel I, mit wertvollen pharmazeutischen Eigenschaften aufzufinden und herzustellen.

Erfindungsgemäß erhält man die neuen 5-(Phenoxyalkanoylamino)-uracile der allgemeinen Formel I indem man entweder

1. 5-Amino-uracile der allgemeinen Formel II

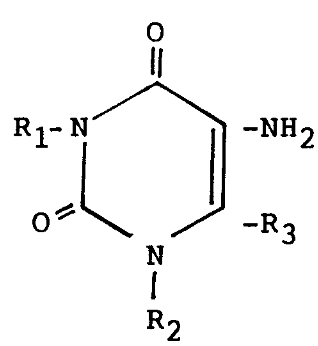

worin $R_1$, $R_2$ und $R_3$ die oben genannte Bedeutung besitzen, mit Phenoxyacetylchloriden oder Phenoxypropionylchloriden bzw. einem reaktionsfähigen Ester einer Phenoxyessigsäure oder Phenoxypropionsäure in an sich bekannter Weise umsetzt oder

2. 5-(Phenoxyalkanoylamino)-uracile der allgemeinen Formel III

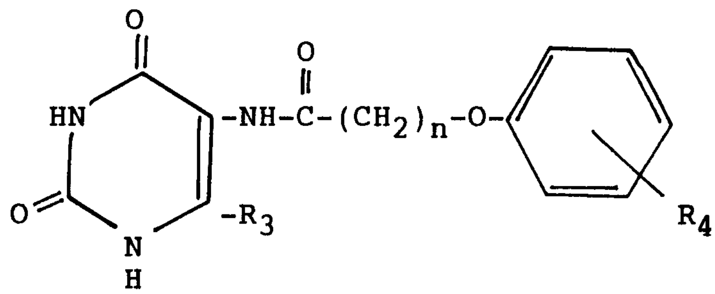

worin $R_3$ und $R_4$ die oben genannte Bedeutung besitzen, durch Alkylierung in die Zielverbindung überführt

oder

3. 5-($\omega$-Halogenalkanoylamino)-uracile der allgemeinen Formel IV

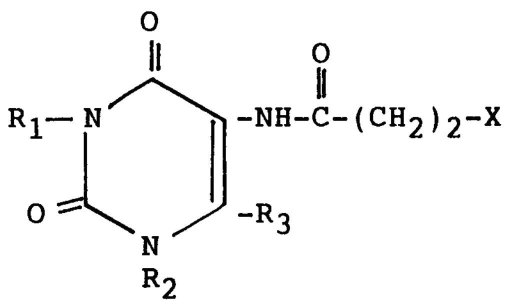

worin $R_1$, $R_2$, $R_3$ und n die oben genannte Bedeutung haben und X ein Chlor oder Bromatom bedeuten, mit Phenolen umsetzt.

Für die Herstellung der neuen 5-(Phenoxyalkanoylamino)-uracile der allgemeinen Formel I nach dem Verfahren Ziffer I können als Lösungsmittel aromatische Kohlenwasserstoffe, wie Benzol, Toluol oder xylol, halogenierte aliphatische oder aromatische Kohlenwasserstoffe, wie Dichlormethan, Chloroform oder Chlorbenzol, Ether, wie Dioxan, Ester, wie Essigsäureethylester , Ketone, wie Aceton, oder Formamide, wie Dimethylformamid eingesetzt werden.

Zur Beschleunigung der Umsetzung ist es vorteilhaft, Halogenwasserstoffakzeptoren, wie Pyridin, Triethylamin, N,N-Dimethylanilin oder Alkalihydrogencarbonate bzw. Alkalicarbonate zu verwenden.

Die Reaktionstemperatur kann in einem größeren Bereich variiert werden.

Im allgemeinen arbeitet man bei der Siedetemperatur des jeweiligen Lösungsmittels.

In Dimethylformamid führt man die Reaktion vorzugsweise bei 30 - 50° C durch.

Die Umsetzung der 5-Amino-uracile der allgemeinen Formel II mit einem reaktionsfähigen Ester kann sowohl in Gegenwart eines inerten organischen Lösungsmittels als auch im Überschuß des jeweiligen Esters erfolgen.

Die Umsetzung wird vorzugsweise bei 150 - 200° C durchgeführt.

Die Ausgangsstoffe der allgemeinen Formel I können entweder durch Alkylierung, Nitrierung und Reduktion von Uracilderivaten der allgemeinen Formel V

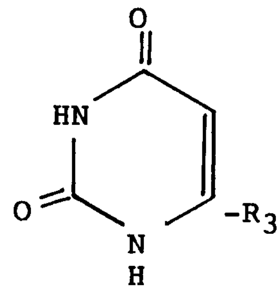

oder aus Uracilderivaten der Formel VI

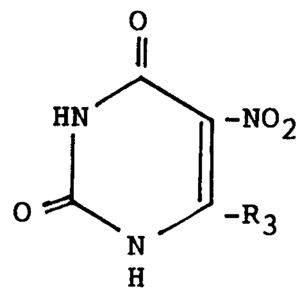

worin $R_3$ die oben genannte Bedeutung besitzt durch Alkylierung und Reduktion nach bekannten Methoden hergestellt werden.

Die Herstellung der erfindungsgemäßen neuen 5-(Phenoxyalkanoylamino)-uracile der allgemeinen Formel I nach Verfahren Ziffer 2 kann in wäßriger, wäßrig-organischer, vorzugsweise in wäßrig-alkoholischer Lösung in Gegenwart von Halogenwasserstoffakzeptoren, wie Alkalihydroxide oder Alkalicarbonate oder in dipolar-aprotischem Lösungsmittel, vorzugweise Dimethylformamid, in Gegenwart von Alkalicarbonaten erfolgen.

Als Alkylierungsmittel eignen sich Dialkylsulfate, Alkylhalogenide, Cycloalkylhalogenide oder Phenylalkylhalogenide.

Die Umsetzung kann in Abhängigkeit von dem jeweiligen Alkylierungsmittel bei Reaktionstemperaturen von 20°C bis zur Siedetemperatur des Lösungsmittels durchgeführt werden.

Die Herstellung der erfindungsgemäßen neuen 5-(Phenoxyalkanoylamino)-uracile der allgemeinen Formel I nach dem Verfahren Ziffer 3 erfolgt in wäßriger, wäßrig-organischer oder organischer Lösung in Gegenwart von Halogenwasserstoffakzeptoren, wie Alkalihydroxide oder Alkalicarbonate bei Reaktionstemperaturen von 20° C bis zur Siedetemperatur des jeweiligen Lösungsmittels oder Lösungsmittelgemisches.

Die Ausgangsverbindung der Formel IV erhält man durch Umsetzung von 5-Aminouracilen der allgemeinen Formel II mit Chlor-oder Bromacetyl bzw. -propionylhalogeniden in einem inerten organischen Lösungsmittel in Gegenwart eines Halogenwasserstoffakzeptors, wie oben angeführt, in an sich bekannter Weise.

Bevorzugt wird die Reaktion in Dimethylformamid bei Temperaturen von 50 - 80° C durchgeführt.

Einen weiteren Bestandteil der vorliegenden Erfindung stellt die pharmazeutische Zubereitung dar.

Sie erfolgt mit einem Gehalt an mindestens einer Verbindung der allgemeinen Formel I gegebenfalls mit anderen anorganischen oder organischen pharmazeutisch verwendbaren Hilfsstoffen.

Eine besondere Ausführungsform besteht in der pharmazeutischen Zubereitung zur Behandlung von Hirnfunktionsstörungen mit einem Gehalt an mindestens einer Verbindung der allgemeinen Formel I .

Als pharmazeutische Zubereitungsformen eignen sich Tabletten, Dragees, Kapseln, Suppositorien, Lösungen und Suspensionen mit mindestens einem Gehalt einer Verbindung der allgemeinen Formel I, gegebenfalls mit anderen anorganischen oder organischen pharmazeutisch verwendbaren Hilfsstoffen.

Sie können nach in der pharmazeutischen Praxis allgemein bekannten und üblichen Verfahren, wie Misch-, Granulier-, Dragier- und Lösungsverfahren hergestellt werden.

Die erfindungsgemäßen neuen 5-(Phenoxyalkanoylamino)-uracile der allgemeinen Formel I besitzen starke cerebroprotektive Wirkungen, die sie für die Anwendung als Arzneimittel bei verschiedenen Hirnschädigungen geeignet machen.

Sie schützen das Hirn vor schädigenden Einflüssen und verbessern seine Leistungsfähigkeit.

Überraschend wurden wesentlich höhere antiamnestische und antihypoxische Wirkungen festgestellt als bei den Vergleichssubstanzen.

Ausgehend von Untersuchungen für gedächtnisverbessernde Substanzen wie z.B von Sara, S. und David-Remacle, M.: Psychopharmacologia 36 (1974) 59 oder Hoffmann, W. und Rostock, A.: Pharmazie 38 (1983), 12,869 beschrieben, ist festgestellt worden, daß die antiamnestische Wirkung einiger Verbindungen der allgemeinen Formel I 3 - 100 fach stärker als die von Piracetam oder 10 - 30 fach stärker als die von Meclofenoxat HCl ist (Tabelle).

Die Verbindungen der allgemeinen Formel I normalisieren die Hirnleistung unter pathologischen Bedingungen wie chronischer Alkoholverbrauch und chronische zerebrale Minderdurchblutung.
Solche Pathogenesefaktoren haben Ähnlichkeit zu Hirnleistungsstörungen des Menschen.
Von besonderer therapeutischer Bedeutung ist der Schutz des Hirns vor Sauerstoffmangel, als eine häufige Ursache von Hirnfunktionsstörungen. Die antihypoxische Wirkung ist darum ein wichtiges Kriterium für die cerebroprotektive Wirkung einer Substanz. Die Verbindungen der allgemeinen Formel I schützen Mäuse in einer Sauerstoffmangelatmosphäre vor dem Erstickungstod. Diese Wirkung ist 3 - 10 fach stärker als die von Piracetam und Meclofenoxat HCl (Tabelle).
Die neuen Uracile sind frei von Nebenwirkungen und hochverträglich.
Die $LD_{50}$ liegt bei 800 bis > 1700 mg/kg bei intraperitonaler Applikation an der Maus.

**Tabelle**

i.p Dosis (mg/kg)

| Substanz vom Beispiel | antiamnestische Wirkung/Ratte | antihypoxische Wirkung/Maus | $LD_{50}$ Maus |
|---|---|---|---|
| 4 | 100 | 30 | >1700 |
| 2 | 1 | 100 | 800 |
| 3 | 3 | 300 | >1700 |
| 1 | 30 | >300 | >1700 |
| Piracetam | 100 | 300 | >2000 |
| Meclofenoxat HCl | 30 | 300 | 856 |

Ausführungsbeispiele

Beispiel 1

5-(p-Chlorphenoxy-acetamido)-1,4-dimethyluracil

Zu 20,5 g (0,1 M) p-Chlorphenoxyacetylchlorid in 150 ml Chloroform gibt man unter Rühren 7 g (0,05 M) 5-Amino-1,4-dimethyluracil.
Anschließend tropft man 8 ml (0,1 M) Pyridin zu, rührt das Gemisch 1 Stunde bei 25 - 30° C und eine weitere Stunde unter Rückfluß. Nach dem Abkühlen wird das Gemisch mit einer Lösung von 17 g Kaliumcarbonat in 200 ml Wasser ausgerührt, wobei ein Niederschlag ausfällt. Dieser wird abgesaugt, mit Chloroform gewaschen und getrocknet.
Ausbeute : 7,3 g Fp. 192 - 207° C ( Zers.)
Die Chloroformphase wird abgetrennt, mit 150 ml Wasser ausgeschüttelt und zur Trockne eingeengt. Der Rückstand wird in 10 ml Isopropanol aufgenommen, abgesaugt und getrocknet.
Ausbeute : 4,4 g Fp. 185 - 198° C (Zers.)
Die vereinigten Niederschläge werden aus 44 ml Isopropanol/Dimethylformamid im Verhältnis 1:1 unter

Zusatz von 1g Aktivkohle umkristallisiert. Das Kristallisat wird abgesaugt, mit Isopropanol gewaschen und getrocknet.
Ausbeute : 7,4 g (45,7 % der Theorie) Fp. 202-211°C (Zers.)

| $C_{14}H_{14}ClN_3O_4$ MG 323,7 | | |
|---|---|---|
| | % berechnet | % gefunden |
| C | 51,9 | 52,0 |
| H | 4,4 | 4,35 |
| N | 13,0 | 12,9 |

Beispiel 2

5-(p-Chlorphenoxy-acetamido)-4-methyluracil

60 g (0,3 M) p-Chlorphenoxyessigsäuremethylester, 8,5 g (0,06 M) 5-Amino-4-methyluracil und 1,5 g Ammoniumchlorid werden 4 Stunden bei 200°C am absteigenden Kühler unter Rühren erhitzt. Nach dem Abkühlen auf ca. 50°C werden 156 ml 96 %iges Ethanol zugegeben und die Suspension wird 15 min verrührt. Anschließend wird der Niederschlag abgesaugt, mit Etanol gewaschen und getrocknet.
Ausbeute : 13,1 g (70,5 % der Theorie) Fp. 280-293°C (Zers.)
Das Rohprodukt wird aus 65 ml Dimethylformamid unter Zusatz von 1,3 g Aktivkohle umkristallisiert. Das Kristallisat wird abgesaugt, mit Dimethylformamid und Aceton gewaschen und bei 110°C getrocknet.
Ausbeute : 5,3 g (28,5 % der Theorie) Fp. 290-296°C (Zers.)
Durch Einengen der Mutterlauge erhält man weitere 3,8 g (20,5 % der Theorie) vom Fp. 288 -293°C ( Zers. ).
Die Gesamtausbeute beträgt damit 9,1 g (49 % der Theorie).

| $C_{13}H_{12}ClN_3O_4$ MG 309,7 | | |
|---|---|---|
| | % berechnet | % gefunden |
| C | 50,4 | 50,4 |
| H | 3,9 | 4,0 |
| N | 13,6 | 13,5 |

Beipiel 3

5-(p-Chlorphenoxy-acetamido)-1,3,4-trimethyluracil

10,3 g (0,033 M) 5-(p-Chlorphenoxy-acetamido)-4-methyluracil werden in 70 ml 1N Natronlauge unter Rühren gelöst. Anschließend tropft man im Verlauf von 2 Stunden 9,5 ml Dimethylsulfat bei 30 - 40°C zu, wobei durch Zutropfen von 1N Natronlauge der pH-Wert ständig über 9 gehalten wird. Nach der Zugabe des Dimethylsulfats wird das Reakionsgemisch eine weitere Stunde bei 30 - 35°C gerührt. Der Niederschlag wird abgesaugt, mit Wasser gewaschen und getrocknet. Das Rohprodukt wird mit 100 ml Chloroform zum Sieden erhitzt und ein unlöslicher Rückstand abgesaugt. Die abgekühlte Chloroformlösung wird mit 100 ml 1N Natronlauge ausgeschüttelt, die Chloroformphase abgetrennt und zur Trockne eingeengt.
Der Rückstand wird anschließend aus 2 Teilen Dimethylformamid unter Zusatz von Aktivkohle umkristallisiert. Das Kristallisat wird abgesaugt, mit 96% igem Ethanol gewaschen und bei 80°C getrocknet.
Ausbeute: 6 g (53,8% der Theorie) Fp. 206-209°C (Zers.)

| $C_{15}H_{16}ClN_3O_4$ MG 337,76 | | |
|---|---|---|
| | % berechnet | % gefunden |
| C | 53,3 | 53,4 |
| H | 4,8 | 4,75 |
| N | 12,4 | 12,4 |

Beispiel 4

4-Amino-5-(p-Chlorphenoxy-acetamido)-1,3-dimethyluracil

Zu einer Lösung von 41 g (0,2 M) p-Chlorphenoxyacetylchlorid in 500 ml Chloroform gibt man 18,8 g 4,5-Diamino-1,3-dimethyluracil. Anschließend werden 16 ml (0,2M) Pyridin unter Rühren und Kühlen zugetropft. Das Gemisch wird je 1 Stunde bei Raumtemperatur und unter Rückfluß gerührt. Nach Erkalten des Gemisches wird der Niederschlag abgesaugt, zweimal mit je 30 ml Chloroform und dreimal mit je 50 ml Ethanol gewaschen. Das ethanolfeuchte Produkt wird mit 400 ml Wasser zum Sieden erhitzt, der Niederschlag heiß abgesaugt, mit heißem Wasser gewaschen und getrocknet.
Das Rohprodukt wird aus 200 ml Dimethylformamid unter Zusatz von Aktivkohle umkristallisiert. Das Kristallisat wird abgesaugt, mit Dimethylformamid und Ethanol gewaschen und getrocknet.
Ausbeute: 27 g (80 % der Theorie) Fp. 259-267°C (Zers.)

| $C_{14}H_{15}ClN_4O_4$ MG 338,7 | | |
|---|---|---|
| | % berechnet | % gefunden |
| C | 49,6 | 49,55 |
| H | 4,5 | 4,6 |
| N | 16,5 | 16,5 |

Beispiel 5

5-(p-Chlorphenoxy-acetamido)-4-methyluracil

Zu einer Suspension von 42,2 g (0,3 M) 5-Amino-4-methyluracil in 400 ml Toluol wird unter Rühren eine Lösung von 82 g (0,4M) p-Chlorphenoxyacetylchlorid in 200 ml Toluol zugetropft. Anschließend wird das Gemisch 7,5 Stunden unter starkem Rückfluß erhitzt. Danach wird das Toluol bei Normaldruck weitestgehend abdestilliert. Das restliche Toluol wird nach Zugabe von 1 Liter Wasser azeotrop entfernt. Zu dem Gemisch gibt man 500 ml Ethanol, erhitzt 30 Minuten unter Rückfluß, saugt den Niederschlag heiß ab, wäscht das Produkt zweimal mit je 100 ml heißem Wasser und 100 ml Ethanol und trocknet es bei 80°C .
Ausbeute: 75 g (80,7 % der Theorie) Fp. 287-293°C (Zer.)
Zur Reinigung wird das Produkt aus 450 ml Dimethylformamid unter Zusatz von Aktivkohle umkristallisiert. Das Kristallisat wird mit Dimethylformamid und Aceton gewaschen und bei 120°C getrocknet.
Ausbeute: 39,8 g (42,8 % der Theorie) Fp. 288-298°C (Zers.)

Beispiel 6

5-(p-Chlorphenoxy-acetamido)-1,3,4-trimethyluracil

1,4 g (0,01 M) p-Chlorphenol, 1,66 g (0,012M) Kaliumcarbonat und 25 ml Dimethylformamid werden 1 Stunde bei 100°C gerührt. Nach Zugabe von 2,45 g (0,01M) 5-Chloracetamido-1,3,4-trimethyluracilwird das Gemisch 3 Stunden bei 100-110°C erhitzt.
Man läßt das Reaktionsgemisch auf ca. 30°C abkühlen, saugt den Niederschlag ab und wäscht ihn mit 10 ml Dimethylformamid. Das Filtrat wird anschließend im Vakuum eingeengt, der Rückstand in 20 ml Methanol suspendiert, abgesaugt, mit Methanol gewaschen und getrocknet.
Ausbeute: 2,16 g (63,7% der Theorie) Fp.157-208°C (Zers.)

Das Rohprodukt wird aus 110 ml Ethanol unter Zusatz von 0,2 g Aktivkohle umkristallisiert.
Ausbeute: 1,7 g (50,3% der Theorie) Fp. 202-208°C (Zers.)
Das Ausgangsprodukt 5-Chloracetamido-1,3,4-trimethyluracil erhält man folgendermaßen:
Zu einer Suspension von 8,5 g (0,05 M) 5-Amino-1,3,4-trimethyluracil in 50 ml Dimethylformamid tropft man 5 ml (0,063 M) Chloracetylchlorid unter Kühlen so zu, daß 35°CInnentemperatur nicht überschritten wird. Das Gemisch wird anschließend 3,5 Stunden bei 30-35°C gerührt. Danach werden ca. 2/3 des Dimethylformamids im Vakuum abdestilliert. Der Rückstand wird abgesaugt, mit Methanol gewaschen und bei 80°C getrocknet.
Ausbeute: 7,7 g (62,7% der Theorie) Fp. 160 -164°C

Beispiel 7

3-Benzyl-5-(p-Chlorphenoxy-acetamido)-1,4-dimethyluracil

13 g (0,04 M) 5-(p-Chlorphenoxy-acetamido)-1,4-dimethyluracil, 8,3 g (0,06M) Kaliumcarbonat und 100 ml Dimethylformamid werden 1 Stunde bei 110°C gerührt. Man tropft 7,6 g (0,06 M) Benzylchlorid zu und erhitzt 4,5 Stunden bei 110-120°C . Nach dem Abkühlen auf Raumtemperatur wird der Niederschlag abgesaugt und mit 10 ml Dimethylformamid gewaschen. Das Filtrat wird im Vakuum eingeengt, der Rückstand in 100 ml Toluol gelöst und die toluolische Lösung mit 50 ml 1N Natronlauge und 50 ml Wasser extrahiert.
Man destilliert das Toluol im Vakuum ab und kristallisiert den Rückstand aus 42 ml Isopropanol unter Zusatz von 1 g Aktivkohle um.
Ausbeute: 11,9 g (71,5% der Theorie) Fp. 118 -123°C

| $C_{21}H_{20}ClN_3O_4$ MG 413,8 | | |
|---|---|---|
| | % berechnet | % gefunden |
| C | 60,9 | 61,0 |
| H | 4,9 | 4,8 |
| Cl | 8,6 | 8,6 |
| N | 10,15 | 10,15 |

Beispiel 8

5-(p-Chlorphenoxyacetamido)-uracil

1,87g (o,01 M) p-Chlorphenoxyessigsäure werden unter Rühren in 30ml getrocknetem Dimethylformamid suspendiert und durch Zugabe von 1,0g (0,01 M) Triethylamin gelöst. Die Reaktionsmischung wird auf 0 - 5°C Innentemperatur abgekühlt, mit 1,1g (0,01 M) Chlorameisensäureethylester versetzt und 1 Minute bei der sich einstellenden Temperatur nachgerührt. Zu der Reaktionsmischung des gemischten Anhydrids gibt man eine Lösung von 1,27g (0,01 M) 5-Aminouracil in getrocknetem Dimethylformamid schnell zu und rührt 30 Minuten nach. Anschließend wird das Reaktionsgemisch in 1 Liter Eiswasser gegossen, der Niederschlag abgesaugt, getrocknet und aus Dimethylformamid zu Wasser im Verhältnis 1:1 umkristallisiert.
Ausbeute: 2,3g ( 78% der Theorie ) Fp > 300°C (Zers.)

| $C_{12}H_{10}ClN_3O_4$ MG 295,7 | | |
|---|---|---|
| | % berechnet | % gefunden |
| C | 48,74 | 48,62 |
| H | 3,41 | 3,59 |
| Cl | 11,99 | 11,98 |
| N | 14,21 | 14,27 |

Beispiel 9

4-Methyl-5-(p-methoxyphenoxyacetamido)-uracil

Hergestellt analog Beispiel 8 aus 1,82g (0,01 M) p-Methoxyphenoxyessigsäure und 1,41 (0,01 M) 5-Amino-4-methyluracil.
Ausbeute: 2,3g ( 75% der Theorie ) Fp. 282 - 284°C ( DMF:H2O = 1:2 )

| $C_{14}H_{15}N_3O_5$ MG 305,3 | | |
|---|---|---|
| | % berechnet | % gefunden |
| C | 55,9 | 54,95 |
| H | 4,95 | 5,33 |
| N | 13,76 | 13,12 |

Beispiel 10

4-Methyl-5-(p-methylphenoxyacetamido)-uracil

Hergestellt analog Beispiel 8 aus 1,66g (0,01 M) p-Methylphenoxyessigsäure und 1,41g (0,01 M) 5-Amino-4-methyluracil.
Ausbeute: 2,2g ( 76% der Theorie ) Fp. 298 - 300°C ( DMF:$H_2O$ = 1:2 )

| $C_{14}H_{15}N_3O_4$ MG 289,3 | | |
|---|---|---|
| | % berechnet | % gefunden |
| C | 58,12 | 58,18 |
| H | 5,23 | 5,24 |
| N | 14,53 | 14,68 |

Beispiel 11

5-(o-Chlorphenoxyacetamido)-4-methyluracil

Hergestellt analog Beispiel 8 aus 1,87g (0,01 M) o-Chlorphenoxyessigsäure und 1,41g (0,01 M) 5-Amino-4-methyluracil.
Ausbeute: 2,2g ( 71% der Theorie ) Fp. 355 - 357°C ( DMF:$H_2O$ = 1:2 )

| $C_{13}H_{12}ClN_3O_4$ MG 309,2 | | |
|---|---|---|
| | % berechnet | % gefunden |
| C | 50,4 | 50,4 |
| H | 3,9 | 3,9 |
| Cl | 11,45 | 11,3 |
| N | 13,6 | 13,7 |

Beispiel 12

5-(p-Chlorphenoxy-β-propionamido)-4-methyluracil

Hergestellt analog Beispiel 8 aus 2,0g (0,01 M) p-Chlorphenoxy-β-propionsäure und 1,41g (0,01 M) 5-Amino-4-methyluracil.
Ausbeute: 2,25g ( 70% der Theorie ) Fp. 235 - 238$^0$C ( DMF:$H_2O$ = 1:1 )

| $C_{14}H_{14}ClN_3O_4$ MG 323,7 | | |
|---|---|---|
| | % berechnet | % gefunden |
| C | 51,94 | 51,99 |
| H | 4,36 | 4,39 |
| Cl | 10,95 | 10,90 |
| N | 12,98 | 12,99 |

Beispiel 13

5-(p-chlorphenoxy-β-propionamido)-1,3,4-trimethyluracil

Hergestellt analog Beispiel 8 aus 2,0g (0,01 M) p-Chlorphenoxy-β-propionsäure und 1,7g (0,01 M) 5-Amino-1,3,4-trimethyluracil.
Ausbeute: 1,4g ( 40% der Theorie ) Fp. 187 - 190$^0$C ( Ethanol)

| $C_{16}H_{18}ClN_3O_4$ MG 351,8 | | |
|---|---|---|
| | % berechnet | % gefunden |
| C | 54,63 | 53,97 |
| H | 5,16 | 5,08 |
| Cl | 10,08 | 10,11 |
| N | 11,94 | 11,99 |

Beispiel 14

5-(p-Chlorphenoxy-β -propionamido)-uracil-hydrat

Hergestellt analog Beispiel 8 aus 1,27g (0,01 M) 5-Aminouracil und 2,0g (0.01 M) p-Chlorphenoxy-β-propionsäure.
Ausbeute: 2,3g ( 70% der Theorie ) Fp. 275 - 278$^0$C ( DMF:$H_2O$ = 1:1 )

| $C_{13}H_{12}ClN_3O$ . $H_2O$ MG 327,7 | | |
|---|---|---|
| | % berechnet | % gefunden |
| C | 47,64 | 47,68 |
| H | 4,31 | 4,36 |
| Cl | 10,82 | 10,76 |
| N | 12,82 | 12,76 |

Beispiel 15

5-(p-Chlorphenoxyacetamido)-3,4-dimethyluracil

12,4g (0,04 M) 5-(p-Chlorphenoxyacetamido)-4-methyluracil werden bei einer Badtemperatur von 120 - 130°C in 16,3g (0,08 M) Bi strimethylsilylacetamid gelöst. Danach werden 20,2g (0,16 M) Dimethylsulfat zugegeben, wobei die Reaktionslösung aufsiedet. Es wird 2 Minuten bei einer Badtemperatur von 120 - 140°C belassen. Nach Abkühlung werden 550 ml Wasser zum Reaktionsge misch gegeben. Der Niederschlag wird abgesaugt, mit Wasser und wenig Methanol gewaschen, getrocknet und aus i-Propanol/ Wasser im Verhältnis 1 : 1 umkristallisiert.
Ausbeute: 1,8g ( 57% der Theorie ) Fp. 267 - 272,5°C

| $C_{14}H_{14}ClN_3O_4$ MG 323,7 | | |
|---|---|---|
| | % berechnet | % gefunden |
| C | 51,94 | 51,76 |
| H | 4,36 | 4,38 |
| Cl | 10,95 | 10,75 |
| N | 12,98 | 12,99 |

**Patentansprüche**

**1.** Neue 5-(Phenoxyalkanoylamino)-uracile der allgemeinen Formel I

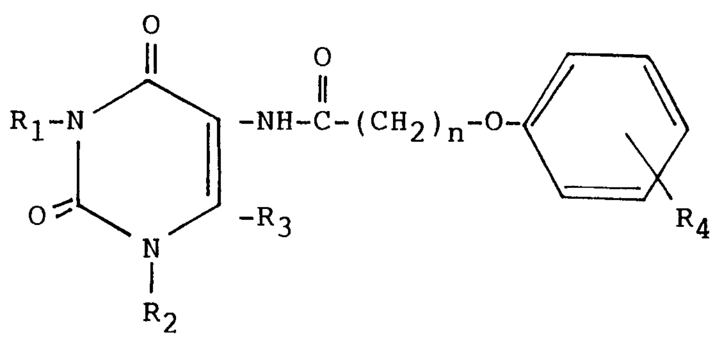

worin

$R_1$ und $R_2$ Wasserstoff, eine Alkylgruppe mit 1-6 C-Atomen, die geradkettig, verzweigt, gesättigt oder ungesättigt oder mit einer Hydroxy-bzw. Oxo-Gruppe substituiert sein kann, eine Cycloalkyl- oder Cycloalkylmethylgruppe, in welcher der Cycloalkylteil jeweils 3 -7 C-Atome enthalten kann, eine Phenylalkylgruppe, in welcher der Alkylteil 1 - 3 Kohlenstoffatome enthalten kann und der Phenylrest durch ein Halogenatom, eine Methoxy-, Methyl- oder Trifluormethylgruppe substituiert sein kann,

$R_3$ Wasserstoff, eine Methyl- oder Aminogruppe,

$R_4$ Halogenatom, Methoxy- oder Methylgruppe,

n 1,2 bedeuten kann.

**2.** Verfahren zur Herstellung von 5-(Phenoxyalkanoylamino)-uracile der Formel I dadurch gekennzeichnet, daß man entweder

a) 5-Aminouracil der allgemeinen Formel II mit Phenoxyacetylchloriden oder Phenoxypropionylchloriden mit einem reaktionsfähigen Ester einer Phenoxyessigsäure oder Phenoxypropionsäure umsetzt oder

b) 5-(Phenoxyalkanoylamino)-uracile der allgemeinen Formel III durch Alkylierung in die Zielverbindung überführt oder

c) 5-(ω-Halogenalkanoylamino)-uracile der allgemeinen Formel IV mit Phenolen umgesetzt.

3. Verfahren nach Anspruch 2a dadurch gekennzeicnet, daß die Umsetzung mit Phenoxyacetyl- bzw. Phenoxypropionylchloriden in einem inerten organischen Lösungsmittel durchgeführt wird.

4. Verfahren nach Ansprüchen 2a und 3 dadurch gekennzeichnet, daß als organisches Lösungsmittel aromatisch Kohlenwasserstoffe, wie Benzol, Toluol oder Xylol, halogenierte aliphatische oder andere aromatische Kohlenwasserstoffe wie Dichlormethan, Chloroform oder Chlorbenzol, Ether wie Dioxan, Ester wie Essigsäureethylester, Ketone, wie Aceton oder Formamide, wie Dimethylformamid eingesetzt werden.

5. Verfahren nach Ansprüchen 2a und 3 dadurch gekennzeichnet, daß die Umsetzung in Gegenwart von Halogenwasserstoffakzeptoren, wie Pyridin, Triethylamin, N,N-Dimethylanilin,Alkalicarbonate oder Alkalihydrogencarbonate erfolgt.

6. Verfahren nach Anspruch 2a dadurch gekennzeichnet, daß die Umsetzung mit einem reaktionfähigen Ester einer Phenoxyessigsäure bzw. Phenoxypropionsäure sowohl in Gegenwart eines inerten organischen Lösungsmittels als auch im Überschuß des jeweiligen Esters durchgeführt wird.

7. Verfahren nach Ansprüchen 2a, 3 bis 5 dadurch gekennzeichnet, daß die Umsetzung mit Phenoxyacetyl- bzw. Phenoxypropionylchloriden vorzugsweise bei der Siedetemperatur des jeweiligen Lösungsmittels durchgeführt wird.

8. Verfahren nach Ansprüchen 2a und 6 dadurch gekennzeichnet, daß die Reaktion mit einem Ester der einer Phenoxyessigsäure bzw. Phenoxypropionsäure vorzugsweise bei Temperaturen von 150 - 200°C erfolgt.

9. Verfahren nach Ansprüchen 2b und 2c dadurch gekennzeichnet, daß die Umsetzung in wäßriger, wäßrig-organischer Lösung der dipolar-aprotischem Lösungsmittel erfolgt.

10. Verfahren nach Ansprüchen 2b, 2c und 9 daduch gekennzeichnet, daß als Lösungsmittel vorzugsweise Alkohol-Wassergemische oder Dimethylformamid verwendet werden.

11. Verfahren nach Ansprüchen 2b, 2c, 8 und 9 dadurch gekennzeichnet, daß die Umsetzung in Gegenwart von Halogenwasserstoffakzeptoren, wie Alkalihydroxiden oder Alkalicarbonaten erfolgt.

12. Verfahren nach Ansprüchen 2b, 2c, 8 bis 11 dadurch gekennzeichnet, daß die Umsetzung bei Temperaturen von 20°C bis zur Siedetemperatur des jeweiligen Lösungsmittels oder Lösungsmittelgemisches durchgeführt wird.

13. Pharmazeutische Zubereitungen gekennzeichnet dadurch, daß sie mindestens einer Verbindung der allgemeinen Formel I enthalten.

14. Verfahren zur Herstellung von pharmazeutischen Zubereitungen gemäß Anspruch 13 dadurch gekennzeichnet, daß man mindestens eine Verbindung der allgemeinen Forml I aus Anspruch 1 mit physiologisch verträglichen anorganisch oder organisch pharmazeutisch geeigneten Hilfsstoffen in eine pharmazeutische Zubereitungsform bringt.

15. Verwendung der Verbindungen der allgemeinen Formel I nach Anspruch 1 als Arzneimittel zur Behandlung von Hirnfunktionsstörungen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 91115447.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CH - A - 484 155 (HOECHST) * Anspruch 1; Spalte 4, Zeilen 24-31 * | 1,2, 13-14 | C 07 D 239/54<br>A 61 K 31/505 |
| A | DE - A - 2 819 629 (CASSELLA) * Ansprüche 1,21-24 * | 1,2, 13-14 | |
| D,A | DD - A - 262 367 (INSTITUT) * Anspruch 1 * | 1, 13-14 | |
| A | EP - A - 0 237 289 (TAKEDA) * Seite 1, Zeilen 6-19; Seite 4, Zeile 5 - Seite 7, Zeile 2 * | 1,2, 13-14 | |

| RECHERCHIERTE SACHGEBIETE (Int. Cl.5) |
|---|
| C 07 D 239/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-14

Unvollständig recherchierte Patentansprüche: -

Nicht recherchierte Patentansprüche: 15

Grund für die Beschränkung der Recherche:

Verfahren zur therapeutischen Behandlung des menschlichen oder tierischen Körpers; Art. 52(4) EPÜ

| Recherchenort | Abschlußdatum der Recherche | Prüfer |
|---|---|---|
| WIEN | 14-01-1992 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument